# FASCICULE DE BREVET EUROPEEN

(11) **EP 0 603 089 B1**
(45) Date de publication et mention de la délivrance du brevet: **17.06.1998**
(21) Numéro de dépôt: 93420497.5
(22) Date de dépôt: 13.12.1993
(51) Int. Cl.: A61B 19/00, G01B 11/02

(54) **Procédé de détermination du point d'ancrage fémoral d'un ligament croisé de genou**
Verfahren zur Bestimmung des Femurverankerungspunkts des Kreuzbandes eines Kniegelenks
Procedure for determining the femoral anchorage part of a cruciate knee ligament

(30) Priorité: 15.12.1992 FR 9215549
(43) Date de publication de la demande: 22.06.1994
(73) Titulaire: UNIVERSITE JOSEPH FOURIER, F-38041 Grenoble Cédex (FR)
(72) Inventeur: Bainville, Eric, F-38000 Grenoble (FR); Cinquin, Philippe, F-38000 Grenoble (FR); Julliard, Rémi, F-38320 Herbeys (FR); Troccaz, Jocelyne, F-38320 Eybens (FR); Lavallée, Stéphane, F-38100 Grenoble (FR); Champlebaux, Guillaume, F-38500 Voiron (FR)
(74) Mandataire: de Beaumont, Michel

(56) Documents cités:
- DE-U- 9 005 819
- INNOVATION ET TECHNOLOGIE EN BIOLOGIE ET MEDICINE (ITBM) vol. 13, no. 4 , 1992 pages 410 - 424 L.ADAMS ET AL 'Operation Aid for Head & Neck Surgeons'
- INNOVATION ET TECHNOLOGIE EN BIOLOGIE ET MEDECINE (ITBM) vol. 13, no. 4 , 1992 pages 375 - 393 P. CINQUIN 'IGOR:Image Guided Optical Robot'

## Description

La présente invention concerne le domaine du repérage de position d'organes de formes complexes exerçant les uns par rapport aux autres des mouvements complexes.

Plus particulièrement, la présente invention vise pour de tels organes à repérer des points d'un organe exerçant des mouvements spécifiques par rapport à un autre organe, par exemple des points d'un organe dont la distance reste invariante lors du déplacement de cet organe par rapport à un autre.

La présente invention trouve des applications dans des systèmes mécaniques complexes où il est pratiquement impossible de déterminer par le calcul le mouvement de certains organes par rapport à d'autres. Elle trouve notamment des applications dans le cas d'organes physiologiques et sera décrite ici dans le cadre d'une application concernant le domaine de la chirurgie et plus particulièrement la chirurgie orthopédique du genou.

Il existe en effet des situations cliniques où il est nécessaire de relier deux organes, en tenant compte de certaines contraintes médicales. C'est en particulier le cas pour la chirurgie du genou, et plus précisément lors de la réfection d'un (des) ligament(s) croisé(s) détruit(s) par un traumatisme.

Cette intervention consiste à remplacer ce(s) ligament(s) par un implant prélevé sur le patient lui-même (généralement aux dépens du tendon rotulien) ou par un ligament artificiel. Pour être efficace, cet implant doit être à la limite de la tension lors de tous les mouvements normaux (flexion-extension-rotation) du genou. S'il est trop tendu, il cassera rapidement ; s'il ne l'est pas assez, il n'interdira pas les mouvements anormaux. La contrainte médicale se traduit donc dans ce cas par une contrainte d'isométrie : le ligament doit garder une longueur constante lors de mouvements de flexion-extension.

Pour cela, il est nécessaire de le positionner de telle sorte que la distance entre ses points d'ancrage fémoral et tibial reste constante. Pour déterminer ces points, le chirurgien dispose de connaissances anatomo-physiologiques, qu'il peut compléter par divers dispositifs. Le point tibial est en principe facile à voir, tant en chirurgie conventionnelle qu'en arthroscopie car il est bien dégagé et car le chirurgien sait assez précisément où le placer, guidé qu'il est par le bord antérieur du toit de l'échancrure. A noter qu'il a été démontré qu'une certaine variation de son emplacement n'avait d'ailleurs que peu de conséquences sur l'isométrie du ligament. Le point fémoral est par contre très profond dans la gorge de la trochlée, aussi le chirurgien ne peut-il le déterminer visuellement qu'approximativement. Or, il a été démontré qu'une faible variation de sa position pouvait perturber considérablement l'isométrie du ligament. Aussi, pour guider le choix du point fémoral, certains ont proposé de mettre en place un ressort et de vérifier par des mouvements de flexion-extension que les points d'ancrage où est fixé ce ressort permettent de conserver l'isométrie (et donc l'iso-tension) du ressort.

Une fois les points d'ancrage positionnés, il faut pouvoir les viser. En effet, pour permettre une fixation solide du ligament, on l'introduit dans deux tunnels fémoral et tibial. Le tunnel tibial ne pose aucun problème de réalisation, tant en chirurgie conventionnelle qu'en arthroscopie. Le tunnel fémoral peut être réalisé de deux façons : soit de dehors en dedans, soit de dedans en dehors (tunnel borgne). Dans le premier cas (de dehors en dedans), la mèche perce la corticale externe du fémur en étant guidée par un viseur - pas toujours commode d'emploi et pas toujours aussi précis que souhaité - vers le point d'ancrage au niveau de l'échancrure inter-condylienne. La broche doit arriver exactement au point d'ancrage, toute erreur à ce niveau ayant, comme cela a été indiqué plus haut, des conséquences néfastes sur l'isométrie du ligament. Dans le deuxième cas (de dedans en dehors), la mèche attaque l'échancrure au point isométrique.

Les difficultés qui se présentent pour déterminer de façon certaine le point d'ancrage fémoral ainsi que pour être à coup sûr précis dans la technique de forage de dehors en dedans du tunnel fémoral expliquent la difficulté de cette intervention, tant en chirurgie conventionnelle qu'en arthroscopie. Or, cette intervention :
- est fréquente : plusieurs milliers de cas par an en France, ce en raison de l'importance de la gêne fonctionnelle induite par la rupture du ligament croisé antérieur, rupture fréquente car consécutive à des accidents survenant lors de la pratique de sports de masse (ski, football, hand-ball, volley ball...),
- a pour ambition de réduire le pourcentage d'évolution arthrosique de ces genoux traumatisés ; on admet qu'avec les techniques actuelles de plastie ligamentaire, 50 % de ces genoux traumatisés, opérés ou non, poseront, 25 ans après le traumatisme, le problème d'une prothèse totale de genou.

La fréquence et l'enjeu de oette pathologie justifient une amélioration de la technique opératoire dont un des points passe par l'amélioration de l'isométrie du ligament implanté.

De façon générale, un objet de la présente invention est de prévoir un procédé et un système de détermination d'un point invariant d'un organe mobile par rapport à un autre.

Un objet plus particulier de la présente invention est de déterminer la position du point d'attache fémoral d'un ligament croisé.

Un autre objet de la présente invention est de prévoir un procédé d'intervention au niveau du point d'attache préalablement déterminé.

Pour atteindre ces objets, la présente invention prévoit un procédé de détermination de la position d'un point d'un deuxième organe mobile par rapport à un premier organe tel que la distance entre ce point du deuxième organe et un point déterminé du premier organe soit sensiblement invariante lors du déplacement du premier organe par rapport au deuxième. Ce procédé fait usage d'un système de repérage tridimensionnel de triplets d'éléments émetteurs comprenant notamment un pointeur repérable par ce système, et comprend les étapes consistant à lier un premier triplet au premier organe ; pointer avec le pointeur la position du point déterminé du premier organe et repérer cette position par rapport à celle du premier triplet ; pointer avec le pointeur les positions d'un ensemble de deuxièmes points situés dans une zone du deuxième organe où est susceptible de se trouver le point invariant ; calculer les distances entre le premier point et chacun des deuxièmes points ; déplacer le deuxième organe par rapport au premier et calculer les variations desdites distances ; et sélectionner parmi les deuxièmes points celui pour lequel ladite distance est sensiblement invariante.

Selon une variante de la présente invention, celle-ci comprend en outre les étapes consistant à lier un deuxième triplet au deuxième organe et à repérer les positions des deuxièmes points par rapport à ce deuxième triplet.

Selon un mode de réalisation de la présente invention, celle-ci comprend en outre l'étape consistant à afficher sur un écran les projections sur un plan des deuxièmes points.

Dans une application de la présente invention, le premier organe est un tibia et le deuxième organe est un fémur, le point déterminé du tibia étant un premier point d'attache d'un ligament croisé du genou et le point invariant du deuxième organe étant le point d'attache fémoral de ce ligament croisé.

Selon un mode de réalisation de la présente invention, le système de repérage tridimensionnel est un système à photodiodes et caméras.

Dans une application, le procédé selon l'invention est utilisé pour le positionnement d'un outil sur le point invariant, l'outil ou un support de guidage de cet outil est muni d'un triplet d'éléments émetteurs et l'outil ou son support est positionné pour que le point d'action de l'outil passe par le point invariant. L'outil est par exemple une perceuse.

L'invention prévoit aussi un système de mise en oeuvre du procédé ci-dessus.

Ces objets, caractéristiques et avantages ainsi que d'autres de la présente invention seront exposés en détail dans la description suivante d'un mode de réalisation particulier faite en relation avec les figures jointes parmi lesquelles :
la figure 1 représente très schématiquement une articulation tibio-fémorale ; et
la figure 2 représente une vue sur un écran de la projection d'un ensemble de points pointés sur une région spatiale déterminée.

La présente invention sera exposée plus particulièrement dans le cadre de la détermination du point d'attache fémoral d'un ligament croisé de genou.

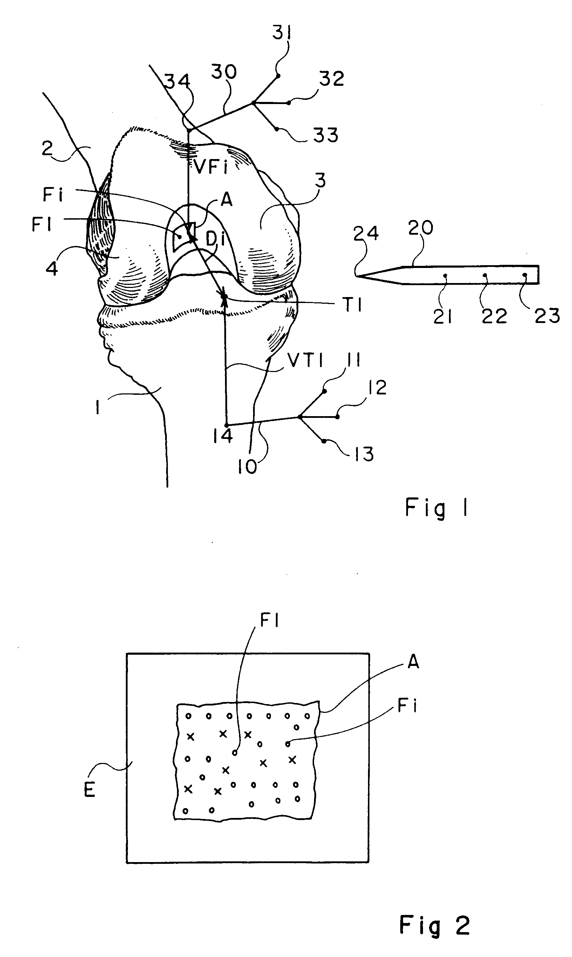

La figure 1 représente très schématiquement un tibia 1 et un fémur 2. Le fémur comprend deux condyles 3 et 4 sur lesquels est articulé le tibia. Le ligament détruit à remplacer doit être situé entre un point d'attache tibial T1 et un point d'attache fémoral F1. Comme on l'a indiqué précédemment, le point d'attache T1 peut être déterminé a priori par le chirurgien à partir d'une observation, tandis qu'en ce qui concerne le point d'attache fémoral, on sait seulement qu'il doit se trouver dans une zone A qui est une portion de la surface de la gorge trochléenne.

On connaît des systèmes de repérage de la position d'émetteurs tels que des émetteurs optiques ou infrarouges, mais qui pourraient aussi être des émetteurs de rayonnement à d'autres longueurs d'onde qui utilisent des ensembles de capteurs et déterminent la position de chacun des émetteurs par triangulation. Un exemple d'une telle installation appliquée à la détermination de la position de la tête est décrit dans l'article du journal Innovation et Technologie en Biologie et Médecine (ITBM), volume 13, N° 4, 1992, L. Adams et al. page 410-424. Il existe également des systèmes commercialisés sous la marque "Optotrak" par la société dite Northern Digital.

L'un des éléments de la présente invention réside dans l'application de tels systèmes au problème posé.

Ainsi, le patient est disposé dans un environnement comprenant des caméras propres à détecter les positions de photoémetteurs. Un premier ensemble ou triplet 10 de photoémetteurs 11, 12, 13 est fixé au tibia en un point 14, par exemple par vissage. Un pointeur 20 muni de photoémetteurs 21, 22 et 23 interagissant avec le système de détection permet de déterminer avec précision toute position prise par sa pointe 24. Ce pointeur est utilisé pour pointer le point T1. Il est alors possible par un système informatique classique de déterminer le vecteur VT1 reliant le point 14 au point T1 et donc de localiser le point T1 pour toute position du tibia.

On utilise ensuite à nouveau le pointeur 20 pour déterminer les coordonnées d'une série de points disposés dans la zone A de la surface de la gorge trochléenne dans laquelle est susceptible de se trouver le point d'attache fémoral recherché F1 et on mémorise la position d'une succession de points Fi pointés.

Pour simplifier la tâche de celui qui réalise l'analyse, il est possible, par des moyens connus associés aux systèmes de localisation spatiale susmentionnés, et comme cela est représenté en figure 2, d'afficher sur un écran E la localisation d'une projection des points Fi sur un plan. Ceci permet en particulier de déterminer si l'on a bien analysé toute la surface que l'on souhaitait pointer. Par exemple, si on a pointé une série de points Fi indiqués par des ronds en figure 2, on s'apercevra facilement sur l'écran qu'il serait souhaitable de pointer des points supplémentaires, marqués dans la figure par des croix, pour réaliser une analyse régulière de la surface A.

Par un traitement informatique classique, on calcule les distances Di entre le point T1 et chacun des points Fi. Ces distances sont mémorisées. Des programmes pour effectuer ces calculs et ceux mentionnés ci-après peuvent être réalisés pour tout spécialiste de la programmation qui pourra, par exemple, s'inspirer de l'article de P. Cinquin et al, "IGOR : Image Guided Operating Robot", ITBM, Vol. 13, N° 4, 1992.

Ensuite, le fémur restant fixe, on fait parcourir au tibia son trajet normal de flexion-extension tout en détectant les variations de position résultantes du triplet 10 et corrélativement du point T1. Simultanément ou ultérieurement, selon les capacités du système de traitement, on calcule les variations de chacune des distances Di entre le point T1 et chacun des points Fi et on sélectionne comme point d'attache fémoral le point F1 parmi les points Fi pour lequel la variation de distance Di a été minimale au cours de ce déplacement.

Dans ce qui précède, on a supposé que le fémur restait fixe. Pour s'affranchir de oette contrainte, selon un mode de réalisation préféré, il est prévu un deuxième triplet 30 de photoémetteurs, comprenant par exemple trois photodiodes 31, 32, 33, fixé au fémur en un point 34. Le triplet 30 peut être localisé par le système détecteur de la même façon que le triplet 10 et le vecteur VFi entre le point 34 et chacun des points Fi peut être calculé. Ainsi, les points Fi sont localisés même si le fémur se déplace.

La présente invention prévoit également d'effectuer une intervention au niveau du point F1 précédemment déterminé, cette intervention étant exactement située au point F1. Pour cela, la présente invention prévoit un outil d'intervention, par exemple une perceuse, portant lui aussi des éléments photoémetteurs pour assurer le positionnement de son point d'action. Les photoémetteurs pourront être prévus ou bien sur l'outil lui-même ou bien sur un guide porte-outil ou bien encore sur un instrument fixant visuellement la position du point d'intervention souhaité, par exemple un laser éclairant le point F1. Ainsi, par exemple, un chirurgien pourra d'abord positionner un guide de façon souhaitée puis enfoncer une perceuse ou bien à partir du point F1 vers l'intérieur ou bien de l'extérieur du condyle vers le point F1 pour ménager un tunnel débouchant au point F1. Ensuite, de la façon indiquée précédemment, un tendon naturel ou artificiel pourra être mis en place dans le tunnel ainsi formé.

La présente invention est susceptible de nombreuses variantes et modifications qui apparaîtront à l'homme de l'art. Notamment, elle pourra être appliquée comme cela a été indiqué précédemment à d'autres domaines qu'au positionnement d'un tendon croisé tibio-fémoral. Egalement, on a prévu précédemment un procédé permettant de sélectionner un point d'un organe tel que sa distance reste invariante par rapport à un point d'un autre organe mobile par rapport au premier. Le point sélectionné pourra, dans d'autres applications, être choisi en fonction de tout autre critère. On pourra par exemple inversement choisir un point présentant un déplacement maximal par rapport à un autre ou encore un point présentant un déplacement par rapport à un autre conforme à une fonction souhaitée.

## Revendications

1. Procédé de détermination de la position d'un point d'un deuxième organe mobile par rapport à un premier organe tel que la distance entre ce point du deuxième organe et un point déterminé du premier organe soit sensiblement invariante lors du déplacement du premier organe par rapport au deuxième, ce procédé faisant usage d'un système de repérage tridimensionnel de triplets d'éléments émetteurs comprenant notamment un pointeur repérable par ce système, caractérisé en ce qu'il comprend les étapes suivantes :
lier un premier triplet (14) au premier organe ;
pointer avec le pointeur (20) la position du point déterminé (T1) du premier organe et repérer cette position par rapport à celle du premier triplet ;
pointer avec le pointeur les positions d'un ensemble de deuxièmes points (Fi) situés dans une zone (A) du deuxième organe où est susceptible de se trouver le point invariant ;
calculer les distanoes (Di) entre le premier point et chacun des deuxièmes points ;
déplacer le deuxième organe par rapport au premier et calculer les variations desdites distances ; et
sélectionner parmi les deuxièmes points celui (F1) pour lequel ladite distance est sensiblement invariante.

2. Procédé selon la revendication 1, caractérisé en ce qu'il comprend en outre les étapes consistant à lier un deuxième triplet (30) au deuxième organe et à repérer les positions des deuxièmes points par rapport à ce deuxième triplet.

3. Procédé selon la revendication 1, caractérisé en ce qu'il comprend l'étape consistant à afficher sur un écran (E) les projections sur un plan des deuxièmes points.

4. Procédé selon l'une quelconque des revendications 1 à 3, caractérisé en ce que le premier organe est un tibia (1) et le deuxième organe est un fémur (2), le point déterminé (T1) du tibia étant un premier point d'attache d'un ligament croisé du genou et le point invariant (F1) du deuxième organe étant le point d'attache fémoral de ce ligament croisé.

5. Procédé selon la revendication 1, caractérisé en ce que le système de repérage tridimensionnel est un système à photodiodes et caméras.

6. Application du procédé selon l'une quelconque des revendications 1 à 5, au positionnement d'un outil sur le point invariant, caractérisé en ce qu'il consiste à munir l'outil ou un support de guidage de cet outil d'un triplet d'éléments émetteurs et à positionner l'outil ou son support pour que le point d'action de l'outil passe par ledit point invariant.

7. Application du procédé selon les revendications 4 et 6 à la réalisation d'un perçage passant par le point invariant du deuxième ensemble de points, caractérisé en ce qu'il consiste à munir un support de guidage d'un outil de perçage d'un triplet d'éléments émetteur et à positionner ce support pour que l'axe de perçage passe par le point invariant.

8. Système de détermination de la position d'un point d'un deuxième organe mobile par rapport à un premier organe tel que la distance entre ce point du deuxième organe et un point déterminé du premier organe suive une loi prédéterminée lors du déplacement du premier organe par rapport au deuxième, ce système comprenant des moyens de repérage tridimensionnel de triplets d'éléments émetteurs et d'un pointeur, caractérisé en ce qu'il comprend :
un premier triplet (14) fixé au premier organe ;
un pointeur (20) pour pointer la position du point déterminé (T1) du premier organe et les positions d'un ensemble de deuxièmes points (Fi) situés dans la zone du deuxième organe où est susceptible de se trouver le point recherché ; et
des moyens de calcul de la position du point déterminé par rapport à celle du premier triplet, des distances (Di) entre le premier point et chacun des deuxièmes points, et des variations desdites distances quand le deuxième organe se déplace par rapport au premier ;
d'où il résulte que l'on peut sélectionner parmi des deuxièmes points celui (F1) pour lequel ladite distance suit ladite loi prédéterminée.

9. Système selon la revendication 8, caractérisé en ce qu'il comprend en outre un deuxième triplet (30) lié au deuxième organe et des moyens de calcul des positions des deuxièmes points par rapport à ce deuxième triplet.

## Claims

1. A method for determining the position of a point of a second organ movable with respect to a first organ, such that the distance separating said point of the second organ from a predetermined point of the first organ is substantially invariant during the movement of the first organ with respect to the second organ, said method using a three-dimensional locating system including triplets of emitting elements especially including a pointer that can be located by said system, characterized in that it includes the following steps:
linking a first triplet (14) to the first organ;
pointing with the pointer (20) at the position of the predetermined point (T1) of the first organ and locating this position with respect to the position of the first triplet;
pointing with the pointer at the positions of an assembly of second points (Fi) that are positioned within an area (A) of the second organ where the invariant point is likely to be found;
calculating the distances (Di) separating the first point from each of the second points;
moving the second organ with respect to the first organ and calculating the variations in said distances; and
selecting amongst second points the point (F1) for which said distance is substantially invariant.

2. The method of claim 1, characterized in that it further includes the steps consisting in linking a second triplet (30) to the second organ and locating the positions of the second points with respect to said second triplet.

3. The method of claim 1, characterized in that it includes the step consisting in displaying on a screen (E) the projections of the second points on a plane.

4. The method of any of claims 1 to 3, characterized in that the first organ is a tibia (1) and the second organ is a femur (2), the determined tibial point (T1) being a first fixation point of a crossed ligament of the knee, and the invariant point (F1) of the second organ being the femoral fixation point of said crossed ligament.

5. The method of claim 1, characterized in that said three-dimensional locating system is a system including photodiodes and cameras.

6. Application of the method according to any of claims 1 to 5 to the positioning of a tool at the invariant point, characterized in that the tool or a guiding support of said tool is provided with a triplet of emitting elements and wherein the tool or its support is positioned so that the operating point of the tool passes through the invariant point.

7. Application of the method according to claims 4 and 6 to a drilling through the invariant point of the second set of points, characterized in that it consists in providing a guiding support of a drilling tool with a triplet of emitting elements and in positioning said support so that the axis of the drilling passes through the invariant point.

8. A system for determining the position of a point of a second organ movable with respect to a first organ such that the distance between said point of the second organ and a determined point of the first organ follows a predetermined law during the displacement of the first organ with respect to the second organ, said system including three-dimensional locating means including triplets of emitting elements and a pointer, characterized in that it includes:
a first triplet (14) fixed to the first organ;
a pointer (20) to point at the position of the determined point (T1) of the first organ and at the positions of an assembly of second points (Fi) that are located within the area of the second organ where the desired point is likely to be found; and
means for calculating the position of the determined point with respect to the position of the first triplet, distances (Di) between the first point and each of the second points, and variations of said distances when the second organ moves with respect to the first organ;
whereby it is possible to select amongst second points the point (F1) for which said distance follows said predetermined law.

9. The system of claim 8, characterized in that it further includes a second triplet (30) associated with the second organ and means for calculating the positions of the second points with respect to said second triplet.

## Patentansprüche

1. Verfahren zur Bestimmung der Lage eines Punkts eines zweiten relativ bezüglich einem ersten Organ beweglichen Organs, derart daß der Abstand zwischen diesem Punkt des zweiten Organs und einem vorgegebenen Punkt des ersten Organs bei einer Verschiebung bzw. Verstellung des ersten Organs relativ gegenüber dem zweiten Organ invariant ist, wobei bei diesem Verfahren ein System zur dreidimensionalen Lokalisierung bzw. Markierung mit Tripletts von Emitter-Elementen angewandt wird, das insbesondere einen mit diesem System lokalisierbaren Zeiger umfaßt,
**dadurch gekennzeichnet**
daß dieses System die folgenden Schritte bzw. Stufen umfaßt:
mit dem ersten Organ wird ein erstes Triplett (14) verbunden;
mit dem Zeiger (20) wird die Lage des vorgegebenen Punkts (T1) des ersten Organs angezeigt bzw. markiert und diese Lage relativ bezüglich dem ersten Triplett lokalisiert;
mit dem Zeiger werden die jeweiligen Lagen eines Ensembles zweiter Punkte (Fi) angezeigt bzw. markiert, welche in einer Zone (A) des zweiten Organs liegen, von welcher angenommen wird, daß sie den invarianten Punkt enthält;
es werden die Abstände (Di) zwischen dem ersten Punkt und jedem der zweiten Punkte berechnet;
das zweite Organ wird relativ bezüglich dem ersten Organ verschoben bzw. verstellt; und
unter den zweiten Punkten wird derjenige (F1) ausgewählt, für welchen der genannte Abstand im wesentlichen konstant ist.

2. Verfahren nach Anspruch 1, dadurch gekennzeichnet, daß es des weiteren die Verfahrensschritte: Anbringen eines zweiten Tripletts (30) an dem zweiten Organ; und Lokalisieren der Lagen der zweiten Punkte relativ bezüglich diesem zweiten Triplett, umfaßt.

3. Verfahren nach Anspruch 1, dadurch gekennzeichnet, daß es den Verfahrensschritt: Anzeige der Projektionen der zweiten Punkte in einer Ebene auf einem Anzeigeschirm (E), umfaßt.

4. Verfahren nach einem der Ansprüche 1 bis 3, dadurch gekennzeichnet, daß das erste Organ eine Tibia (Schienbein) (1) und das zweite Organ ein Femur (Oberschenkel) (2) ist, und daß der vorgegebene Punkt (T1) der Tibia ein erster Befestigungspunkt eines Kniegelenk-Kreuzbandes ist und der invariante Punkt (F1) des zweiten Organs der femorale Befestigungspunkt dieses Kreuzbandes ist.

5. Verfahren nach Anspruch 1, dadurch gekennzeichnet, daß das dreidimensionale Lokalisierungssystem ein System mit Photodioden und Kameras ist.

6. Anwendung des Verfahrens nach einem der Ansprüche 1 bis 5 zur Positionierung eines Werkzeugs auf den invarianten Punkt, dadurch gekennzeichnet, daß das Werkzeug oder eine Führungshalterung dieses Werkzeugs mit einem Triplett von Emitterelementen versehen und das Werkzeug oder seine Führungshalterung so positioniert wird, daß der Stellort des Werkzeugs durch den genannten invarianten Punkt verläuft.

7. Anwendung des Verfahrens nach den Ansprüchen 4 bis 6, zur Durchführung einer durch den invarianten Punkt der zweiten Gesamtheit von Punkten verlaufenden Bohrung, dadurch gekennzeichnet, daß die Führungshalterung eines Bohrwerkzeugs mit einem Triplett von Emitter-Elementen versehen und diese Führungshalterung so positioniert wird, daß die Achse der Bohrung durch den invarianten Punkt verläuft.

8. System zur Bestimmung der Lage eines zweiten beweglichen Organs relativ bezüglich einem ersten Organ derart, daß der Abstand zwischen diesem Punkt des zweiten Organs und einem vorgegebenen Punkt des ersten Organs bei der Verschiebung bzw. Verstellung des ersten relativ bezüglich dem zweiten Organ einem vorgegebenen Gesetz folgt, wobei dieses System Mittel zur dreidimensionalen Lokalisierung mit Tripletts von Emitter-Elementen und einen Zeiger umfaßt, dadurch gekennzeichnet, daß es umfaßt:
ein an dem ersten Organ fixiertes erstes Triplett (14);
einen Zeiger (20) zum Anzeigen bzw. Markieren der Lage des vorgegebenen Punkts (T1) des ersten Organs und der jeweiligen Lagen eines Ensembles zweiter Punkte (Fi) in derjenigen Zone des zweiten Organs, von welcher angenommen wird, daß sie den gesuchten Punkt enthält; sowie
Mittel zum Berechnen der Lage des vorgegebenen Punkts relativ bezüglich der Lage des ersten Tripletts, zur Berechnung der Abstände (Di) zwischen dem ersten Punkt und jeweils jedem der zweiten Punkte, sowie zum Berechnen der jeweiligen Änderungen der genannten Abstände bei einer Verstellung bzw. Verschiebung des zweiten Organs gegenüber dem ersten;
derart daß man unter den zweiten Punkten denjenigen (F1) auswählen kann, für welchen der genannte Abstand dem genannten vorgegebenen Gesetz folgt.

9. System nach Anspruch 8, dadurch gekennzeichnet, daß es des weiteren ein mit dem zweiten Organ verbundenes zweites Triplett (30) sowie Mittel zum Berechnen der jeweiligen Lagen der zweiten Punkte relativ bezüglich dem zweiten Triplett aufweist.
